# EUROPEAN PATENT APPLICATION

(11) **EP 0 738 734 A1**
(43) Date of publication of application: **23.10.1996**
(21) Application number: 95902994.3
(22) Date of filing: 20.12.1994
(51) Int. Cl.: C07K 14/435, C12P 21/02

(54) **NOVEL RECOMBINANT DerI ALLERGENIC SUBSTANCE, PRECURSOR THEREOF, AND PROCESS FOR PRODUCING THEM**

(30) Priority: 22.12.1993 JP 325061/93
(71) Applicant: ASAHI BREWERIES, LTD., Tokyo 104 (JP); TORII PHARMACEUTICAL CO., LTD., Tokyo 103 (JP); THE NIKKA WHISKY DISTILLING CO., LTD., Tokyo 107 (JP)
(72) Inventor: SYOJI, Hiroshi The Nikka Whisky Distilling Co.,, 967, Aza-Matsuyama Masuo Kashiwa-city (JP); SHIBUYA, Ichiro The Nikka Whisky Distilling Co.,, Aza-Matsuyama Masuo Kashiwa-city (JP); OKUMURA, Yasushi Asahi Breweries, Ltd. Central, Tokyo143 (JP)
(74) Representative: Perry, Robert Edward
(86) International application number: JP9402144
(87) International publication number: WO9517424

(57) **Abstract**

A recombinant allergen and a precursor thereof both originating from *Dermatophagoides farinae* or *Dermatophagoides pteronyssinus*s are provided. The recombinant allergen has an allergic activity comparable with that of natural allergen.

The precursor is produced by genetic recombination of *Der* I gene by employing a nuclear polyhedrosis virus as the vector and cells derived from *Spodoptera frugiperda* as the host.

The recombinant allergen is produced by bringing the pH of the solution of the precursor to an acidic side. The recombinant allergen is also produced by inducing site-specific variation in a DNA molecule for encoding a *Der* I allergenic substance, and reactng the resulting molecule with a site-specific protease.

## Description

### TECHNICAL FIELD

The present invention relates to a novel allergenic substance, a precursor thereof, and a process for production thereof. More specifically, the present invention relates to a precursor of a recombinant *Der* I allergen, which precursor originates from *Dermatophagoides farinae*, *Dermatophagoides pteronyssinuss*, or the like and can be secreted from cells, and relates also to a recombinant *Der* I allergen which is derived from the precursor. The present invention further relates to a process for producing them.

### BACKGROUND ART

An increasing number of people are suffering from allergic diseases in recent years. The allergic diseases typically includes pollinosis, atopic dermatitis, and atopic asthma. In particular, the atopic asthma is caused frequently by house dust. The house dust is known to contain mites, spores of molds, dander, and other various matters.

The substance which causes allergic diseases is called allergen. In the above dust, the most important allergen is known to be proteins produced by mites. The mites which scatter the allergen in house include *Dermatophagoides farinae* (hereinafter referred to as "*Der f*"), and *Dermatophagoides pteronyssinuss* (hereinafter referred to as "*Der p*"). Of the mites, *Der f* are known to be dominant mites in Japan.

The particularly important allergens produced by the mites include substances of group I (*Der* I) which are usually contained in the excrement and have a molecular weight of about 25 kDa, and substances of group II (*Der* II) which are mainly contained in the mite body and have a molecular weight of about 14 kDa. They are further classified respectively into *Der f* I and *Der f* II, and *Der p* I and *Der p* II according to the kinds of the mites. The two groups of allergens are known to be reactive to most of the allergic diseases patients (Heymann, et al.: J. Allergy Clin. Immunol. 83, 6, 1055-1067, 1989).

The above allergens have already been isolated in pure forms from the natural products (Yasueda, et al.: Int. Arch. Allergy Appl. Immunol. 88, 402-407, 1989). The genes for encoding them were cloned as cDNA (Dilworth, et al.: Clinical and Experimental Allergy, 21, 25-32, 1991; Chua, et al.: The Rockfeller University Press, 167, 175-182, 1988; Yuuki, et al.: Jpn. J. Allergol., 39, 557-561, 1990, and Chua, et al.: Int. Arch. Allergy Appl. Immunol., 91, 118-123, 1990). From these results, *Der f* I and *Der f* II, and *Der p* I and *Der p* II are confirmed respectively to be highly homologous to each other (Dilworth, et al.: Clinical and Experimental Allergy, 21, 25-32, 1991; Trundinger, et al.: Clinical and Experimental Allergy: 21, 33-37).

International Patent Laid-Open Publication 92/04445 discloses base sequences of the cDNAs for encoding the above allergens, and the predicted sequences. However, the allergens have not been obtained in the biological active form. Therefore, the allergenic activity thereof is not obvious.

Further, the Group-I allergens are known to be a cysteine protease (Ino, et al.: Int. Arch. Allergy Appl. Immunol. 89, 321-326, 1989).

Generally, the allergic diseases is known to be developed by any of four mechanisms, and allergic diseases caused by allergen belongs to Type-I allergy. In this type of allergy, an allergen, namely an antigen, firstly enters a human body in various manners to cause sensitization. Thereby, an IgE antibody specific to this allergen is produced, and the produced IgE antibody combines with mast cells or basophils in the trachea, the digestive tract, the nose mucosa, the skin, or the like. When the allergen is again introduced therein, the allergen causes an antigen-antibody reaction with the specific IgE antibody, whereby a chemical mediator such as histamine is released from the mast cells or basophils and allergic diseases is caused.

The allergy which is developed by this mechanism is called immediate allergy, and the atopic asthma is caused usually by this Type-I allergy.

At the moment, various therapies are employed for the allergic diseases. However, most of them are a symptomatic therapy. In contrast thereto, a hyposensitization is known which has long been conducted for treatment of urushi (Japanese lacquer) craftsmen. In this hyposensitization, an allergen is introduced to the human body in small doses such that the immunity against allergy is acquired without causing allergic diseases.

Therefore, the atopic asthma may possibly be treated effectively by hyposensitization employing the two kinds of allergens produced by mites.

In practicing the hyposensitization, the allergen itself is required in a large quantity. However, the allergen, which exists in only a small amount in nature, can be isolated at a very high cost, and therefore the therapy cannot be applied to many patients. From the same reasons, the collection of fundamental medical data when using the allergen is limited significantly. Accordingly, a technique is demanded which makes available the allergen at a lower cost in a larger amount.

To meet the above demand, genetic recombination technique has come to be employed in recent years; and the group II allergens among the above allergens has been developed by utilizing *Escherichia coli* (*E. coli*) and has approximately the same allergic activity as the natural product (Yuuki, et al.: Int. Arch., Allergy Appl. Immunol., 94, 354-356, 1991; Chua, et al.: Int. Arch., Allergy Immunol., 91, 118-123, 1990).

On the other hand, an allergen similar to Group-I allergen (*Der f* I) was developed by utilizing *E. coli*, but it exhibited little allergic activity (Thomas, et al.: Int. Arch., Allergy Appl. Immunol.,85, 127-129, 1988) and has no practical application.

A recombinant *Der p* I (*Der p* I is highly homologous to *Der f* I) was derived by utilizing an yeast, and was observed to have an allergic activity nearly the same as natural product (Chua, et al.: J. Allergy Clin. Immunol., 95-102, 1992). The developed protein was a precursor protein (hereinafter referred to as "precursor") containing a prosequence, and had a higher molecular weight than the natural product, and was insoluble.

Therefore, for application of this developed precursor to practical therapy or the like, the precursor is required to be solubilized by use of urea, to be desalted, and to be purified by use of a monoclonal antibody. With such troublesome operations, the purified product could be obtained only in a yield of as low as 1 mg per liter of the obtained liquid culture medium (a precursor-containing solution).

Moreover, the purification employing a monoclonal antibody is costly in industrial production.

### DISCLOSURE OF THE INVENTION

The present invention has been accomplished to solve the problems involved in the prior art techniques, and intends to provide a novel recombinant *Der* I allergen precursor which can be secreted from cells and is solubilized, to provide a novel recombinant *Der* I allergenic substance having the same level of allergic activity as the natural product, and to provide a process for producing these at a high yield.

After comprehensive investigation, it was found by the inventors of the present invention that the above problems could be solved by conducting genetic recombination in insect cells and applying, as necessary, a specific post-treatment. Thus the present invention has been completed.

The recombinant *Der f* I allergenic substance of the present invention is characterized by being composed of one or more proteins selected from the group of proteins having an amino acid sequence represented by Sequence No. 1 or Sequence No. 2 of Sequence Table.

The method for producing the allergic substance of the present invention is characterized in that the recombinant *Der* I allergenic substance is produced by expressing *Der* I genes by a genetic recombination technique in insect cells and changing the pH of the resulting solution of recombinant *Der* I allergen precursor to an acidic side.

Further, the precursor of the allergenic substance of the present invention is characterized in that it is constituted of one or more proteins selected from the group of proteins having an amino acid sequence represented by Sequence Nos. 5, 6, 7, or 8 of Sequence Table.

Furthermore, the process for producing the precursor of the allergenic substance of the present invention is characterized in that the precursor of the above recombinant *Der f* I allergenic substance is produced by expressing *Der f* I genes in insect cells by a genetic recombination technique.

Furthermore, another process for producing the recombinant allergenic substance of the present invention is characterized in that
site-specific mutagenesis is introduced in a DNA molecule for encoding the *Der* I allergenic substance, to form a DNA molecule of *Der* I having a recognition sequence for a site-specific protease,
the DNA molecule is expressed in insect cells to form a precursor of the *Der* I allergenic substance having the above recognition sequence, and then
a corresponding site-specific protease is allowed to act on the formed precursor.

Of the *Der* I allergens relating to the present invention, a pre-precursor of the recombinant *Der f* I allergenic substance is described below.

The pre-precursor is a protein having an amino acid sequence represented by Sequence No. 3 or 4 in Sequence Table, and has a pre-pro-sequence as shown in the same Sequence Number. Any mixture of the protein of Sequence No. 3 and the protein of Sequence No. 4 is also included in the pre-precursor.

Next, the precursor of the recombinant *Der f* I allergenic substance of the present invention is described below.

The precursor of the present invention is a protein having an amino acid sequence represented by any of Sequence Nos. 5-8 of Sequence Table, and has a pro-sequence corresponding to the same Sequence Number. Any mixture composed of combination of the proteins selected from Sequence Nos. 5, 6, 7, and 8 is included in the precursor of the present invention.

By the action of the pre-pro-sequence shown by Sequence No. 3 or 4, the allergen precursor of the present invention is secreted in a dissolved state to the outside of the cell.

As to the relation between the pre-pro-sequence and the pro-sequence both according to the present invention, the pro-sequence is presumed to promote the action of pre-sequence for the secretion.

When a protein has only a pre-sequence, it is thought that the protein is secreted to the outside of the cell only in a very small amount, or, in some cases, it is not secreted at all. On the contrary, a protein having a pro-sequence in addition to a pre-sequence, the protein (precursor) is secreted in a remarkably large amount.

Any of the precursors of the present invention has a significant allergic activity, although the allergic activity is lower than that of natural *Der f* I allergen. However, the precursor of the present invention is advantageous in that an allergic activity is obtained which has been unobtainable with the recombinant *Der f* I allergen produced by using *E. coli* and that the precursor is obtained in a solution differently from the recombinant *Der p* I allergen produced by utilizing a yeast.

The term "allergic activity" in the present specification means an activity which causes an antigen-antibody reaction with an IgE antibody.

According to the present invention, the pre-sequence and pro-sequence of *Der f* I allergen has been determined from the precursor, and these sequences can be produced by utilizing insect cells as described later, whereby the following matters are made possible.
(1) The above pre-sequence is inserted into a vector to be used in an expression system using insect cells and the resulting vector can be used as a vector for secretion and expression. In other words, the *Der f* I pre-sequence is fused with a different gene and the fusion product can be secreted and expressed in insect cells.
(2) The above pre-pro-sequence is inserted into a vector to be used in an expression system using insect cells and the resulting vector can be used as a vector for secretion and expression. In other words, the *Der f* I pre-pro-sequence is fused with a different gene and the fusion product can be secreted and expressed in insect cells.

Then, description is made on the method for producing a precursor of the recombinant *Der f* I allergenic substance of the present invention.

The precursor is obtained by using insect cells as a host and, as a vector, a nuclear polyhedrosis virus (NPV) which is one of insect viruses and belongs to Baculoviridae.

Such a method using eucaryotic cells of an insect can increase the production yield, utilizing this effective promoter, and enables the product to be modified by eucaryote in various manners (sugar chain modification, phosphorylation, sialation, processing, and so forth), because the NPV produces the inclusion bodies called polyhedral bodies in infected cell nuclei amount of as much as about 50% of the entire proteins.

The insect cells used in the above method may be any insect cells capable of being infected with nuclear polyhedrosis, including cells of *Lepidoptera*, *Hymenoptera*, and *Diptera*: specifically including cells of BmN (*Bombix mori* N), BoMo, and Bm36 of silk worms; cells of Sf9 and Sf21 of *Spodoptera frugiperda*; and cells of Tn-368 and High Five of *Tichoplusia ni*.

The nuclear polyhedrosis virus also used in the above method is exemplified by AcNPV (Autographa california nuclear polyhedorosis virus) for the Sf9 cells, and BmNPV for the BmN cells.

An example of the operation for production of the allergen precursor of the present invention is described below.

Firstly, the *Der f* I gene is inserted into a cloning site of Bacurovirus transfer vector. The resulting *Der f* I-inserted transfer vector and a DNA of wild type AcNPV are allowed to co-transfect the above Sf9 cell. This co-transfection causes homologous recombination since the *Der f* I-inserted transfer vector has, on its both sides of the cloning site, the same base sequence as that of the wild type AcNPV DNA, whereby a recombinant virus is produced. The resulting recombinant virus is separated selectively from the culture supernatant by a plaque technique. The obtained recombinant virus is allowed to infect the Sf9 cells to produce a precursor of the *Der f* I allergen in the culture supernatant or in the Sf9 cells.

The allergen precursor of the present invention is obtained as above in a solution state (culture supernatant), so that the solubilization with a solubilizer, or desalting by dialysis for purification usually conducted is not required, and the problems such as refolding and the like need not be considered.

Accordingly, the allergen precursor of the present invention can readily be obtained from the precursor-containing solution by a usual simple operation such as water removal or the like. Therefore, the recombinant *Der f* I allergen precursor of the present invention is obtained at a high yield of about 50 mg per liter of the precursor-containing solution.

The insoluble precursor existing in the above Sf9 cells can be solubilized readily by use of a surfactant such as Triton X-100 (produced by Nakarai Tesuku K.K.) or the like.

Next, the recombinant *Der f* I allergenic substance of the present invention is described below.

The recombinant *Der f* I allergenic substance of the present invention is a protein having an amino acid sequence represented by Sequence No. 1 or 2 of Sequence Table, including mixtures of proteins of Sequence No. 1 and Sequence No. 2.

In the case where the recombinant *Der f* I allergenic substance is obtained in the form of a mixture, the mixture may be separated into the components allergenic substances by, for example, a SDS-PAGE technique (sodium dodecylsulfate-polyacrylamide gel electrophoresis), an HPLC technique (high performance liquid chromatography), or a monoclonal antibody treatment.

The recombinant *Der f* I allergenic substance can be obtained by adjusting the pH of the above solution of the allergen precursor of the present invention to be in an acidic range, preferably at pH=3-4, more preferably pH=4. Since the allergenic substance can be obtained by such a simple treatment of the precursor and, as mentioned above, the precursor is obtained in a high yield, the allergenic substance of the present invention can also be produced at a remarkably higher yield and in a remarkably larger amount than in conventional production process.

The pH adjustment causes cleavage of the pro-sequence of the precursor of the present invention to give a recombinant *Der f* I allergenic substance having an allergenic activity nearly the same as that of natural *Der f* I allergen.

The above recombinant *Der f* I allergenic substance can also be produced by causing site-specific variation in a DNA molecule which encodes the *Der f* I allergenic substance, and then reacting the resulting molecule with a site-specific protease.

In this process, site-specific variation is caused in the above DNA by PCR (polymerase chain reaction) or the like to produce a *Der f* I DNA molecule having a recognition sequence for a site-specific protease at the C-terminal codon of pro-sequence. Then, the resulting DNA molecule is expressed in insect cells as mentioned above to produce a precursor of the *Der f* I allergenic substance having the above or recognition sequence. The precursor is reacted with the above site-specific protease to cleave the pro-sequence to produce the recombinant *Der f* I allergenic substance of the present invention.

Table 1 shows the site-specific proteases usable in the above production process, and the recognition sequences thereof.

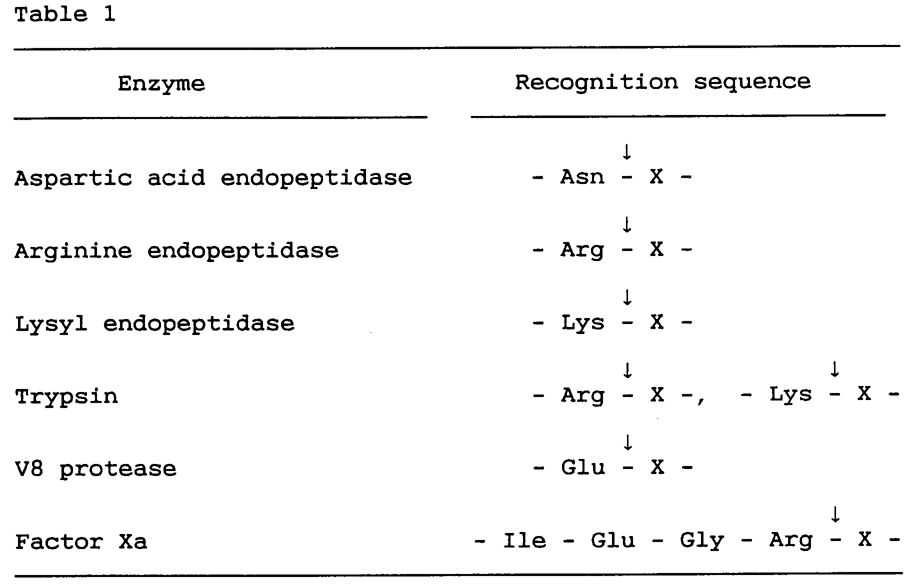

As mentioned above, the present invention can produce, in a large amount, a recombinant *Der f* I allergenic substance having an allergic activity nearly the same as that of the natural *Der f* I allergen. As a result, a hyposensitization therapy, in addition to the conventional symptomatic therapy, has been made usable for the treatment of allergies, particularly atopic asthma.

The *Der f* I allergen is highly homologous with the *Der p* I allergen. Therefore, it is obvious that the above pH adjustment method or site specific variation method is applicable to the *Der p* I allergen.

The amino acid sequence of the recombinant *Der f* I allergenic substance of the present invention can be specified, and the pre-sequence and the pro-sequence of the present *Der f* I allergenic substance can be determined as described above, and further the present *Der f* I allergenic substance can be obtained by pH adjustment. Therefore, the following matters are realized by use of the *Der f* I allergenic substance or the precursor thereof.
(1) The *Der f* I allergenic substance (maturation form) can be secreted and expressed in a different expression system. Specifically, the precursor of the *Der f* I allergen is combined, by fusion, with another vector for secretion and expression, and the pH of the resulting protein solution is adjusted to cleave a bond between pro-sequence and mature sequence to obtain the *Der f* I allergenic substance (maturation form).
(2) The *Der f* I allergenic substance can be developed as a fusion protein with another protein gene. Specifically, the precursor of the *Der f* I allergenic substance is fused with ........., and the resulting protein solution is treated at a desired pH to cleave the pro-sequence from the maturation sequence to obtain the *Der f* I allergenic substance (maturation form).

### BRIEF DESCRIPTION OF THE DRAWINGS

Fig. 1 is a drawing for explaining a method for introducing the *Der f* I gene to a transfer vector.
Fig. 2 is a graph showing RAST activities of recombinant *Der f* I allergens.
Fig. 3 shows change of molecular weight of a *Der f* I allergen precursor with change of pH.
Fig. 4 shows change of molecular weight of another *Der f* I allergen precursor with changes of pH and temperature.
Fig. 5 shows change of molecular weight of a still another *Der f* I allergen precursor with changes of pH and temperature.
Fig. 6 shows change of molecular weight of a still another *Der f* I allergen precursor with changes of pH and temperature.
Fig. 7 shows change of molecular weight of a still another *Der f* I allergen precursor with changes of pH and temperature.
Fig. 8 shows the results of SDS-PAGE analysis of variant pecursors.
Fig. 9 shows the results of SDS-PAGE analysis of recombinant proteins having been treated with a site-specific protease.
Fig. 10 is a graph showing RAST activities of recombinant *Der f* I allergens and other substances.
Fig. 11 shows the results of SDS-PAGE analysis of variant precorsors after pH adjustment.

### BEST MODE FOR PRACTICING THE INVENTION

The present invention is described below in more detail by way of Examples with reference to the accompanying drawings. However, the present invention is not limited to the Examples.

### (1) Introduction of Der f I Gene to Transfer Vector:

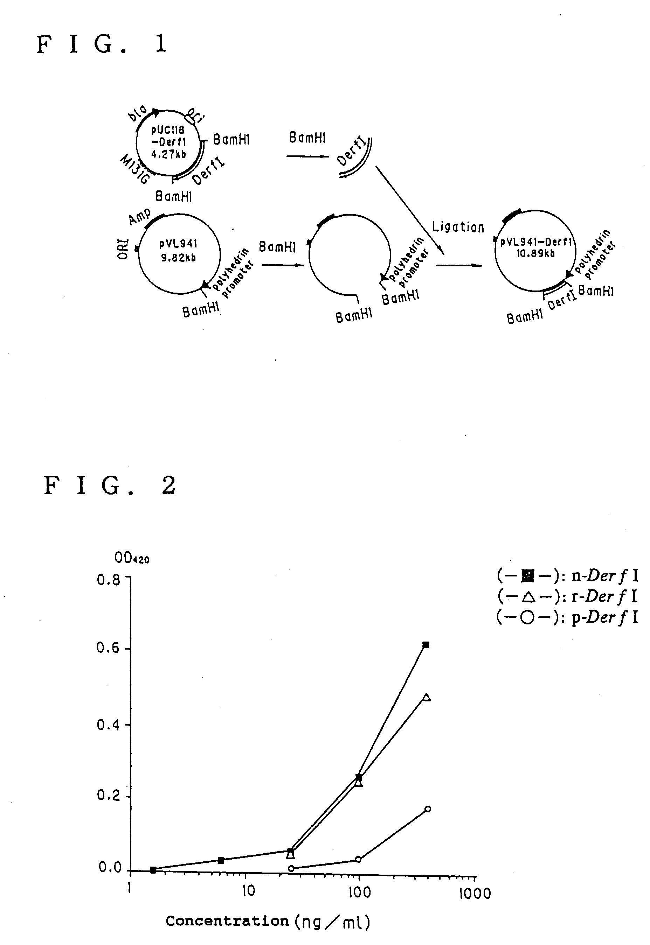

Fig. 1 shows an example of the method of introducing the *Der f* I gene to a transfer vector.

The *Der f* I gene subcloned to Vector pUC118 (the gene had been produced by Taka ra Shuzo Co., Ltd. and received from Yasuhara, Special Edition of 42th General Meeting of Japan Allergology Society, Vol.4, No.8, 162, 1992) was cleaved by a restriction enzyme *Bam*HI (produced by Takara Shuzo Co, Ltd.); the resulting fragment was subjected to agarose electrophoresis; the objective gene fragment was cut out; and elution with DNACELL (produced by Daiichi Kagaku K.K.) was conducted to obtain a *Der f* I gene fragment.

Separately, a transfer vector pVL941 (produced by Pharmingen Co.) was cleaved by the *Bam*HI in the same manner as above. The above *Der f* I gene fragment was inserted into the pVL941 by use of a T4DNA ligase (produced by Takara Shuzo Co., Ltd.). The resulting transfer vector was introduced into competent *E. coli* HB101 (Takara Shuzo Co., Ltd.) to cause transformation. Some colonies were collected selectively to obtain a transfer vector (pVL941-Der f I) in which insertion was made in an intended direction. This transfer vector (pVL941-Der f I) was purified by means of a Qiagen Plasmid Kit (manufactured by Qiagen Co.).

### (2) Incubation of Insect Cells, and Preparation and Purification of Recombinant Virus:

### (i) Preparation of culture medium:

A culture medium was prepared according to the method of Matsuura [Tanpakushitsu, Kakusan, Koso (Proteins, Nucleic Acids, and Enzymes), Vol.37, No.3, 211-222, 1992].

Bovine fetal serum was added in an amount of 10% to TC-100 (produced by Gibco Co.), and thereto was added gentamicin sulfate (antibiotic produced by Wako Pure Chemical Industries, Ltd.) in the final concentration of 50 µg/ml to prepare a culture medium. In place of such a serum-containing culture medium, a non-serum culture medium such as sf900II (produced by Gibco Co.) or the like can be used.

### (ii) Preparation of culture fluid:

In a Petri dish of 60 mm diameter, was placed 3 × 10⁶ cells of Sf9, and thereto was added 3 ml of the culture medium. The Sf9 cells (insect cells) were incubated at a temperature of 27°C with exchange of the culture medium two or three times a week to prepare a culture fluid.

### (iii) Preparation of recombinant virus:

The recombinant virus was prepared by co-transfecting the Sf 9 cells with the above *Der f* I-introduced transfer vector (pVL941-Der f I) and the DNA of wild type AcNPV by using Lipofectin (produced by Gibco Co.).

Specifically, 10 ng of DNA of wild type AcNPV and 1 µg of pVL941-Der f I were dissolved in sterilized water to make the total volume 8 µl. Thereto was added an equal volume of Lipofectin diluted with sterilized water by a factor of 2. The mixture was left standing at room temperature for 15 minutes to obtain a DNA liquid mixture.

Separately, 1 × 10⁶ cells of the above incubated Sf9 was transferred to a Petri dish of 35 mm diameter, and the culture medium was discarded. Thereto was added 1 ml of the above-mentioned no-serum-containing culture medium. The above DNA liquid mixture was added to this Sf9 cells, and the mixture was kept standing at 27°C for 24 hours. Then the culture medium was replaced by the bovine fetal serum-containing culture medium, and incubation was conducted for 4 days. The resulting culture supernatant and/or the Sf9 cells were considered to contain an intended recombinant virus, other viruses and a small amount of an intended recombinant protein.

Therefore, the presence of the intended recombinant protein was confirmed preliminarily by analyzing the obtained culture supernatant and the Sf9 cell extract by Western blotting technique using anti-*Der f* I allergen serum.

For the confirmation, the culture supernatant and the Sf9 cell extract solution were transferred electrically, by use of a 25 mM Tris-192 mM glycine-20% methanol buffer solution, onto a nitrocellulose membrane (produced by Bio-Rad. Co.). The obtained membrane was blocked by a phosphate buffered saline (hereinafter referred to as "PBS") containing 1% bovine serum albumin, and washed with PBS containing 0.05% Tween 20. Then, reaction was conducted with an anti-*Der f* I rabbit antigen diluted by a factor of 10000 with PBS containing 1% bovine serum albumin. The anti-*Der f* I rabbit antibody was obtained by separating and purifying a natural *Der f* I from a freeze dried product of mite culture according to the method of Yasueda (Int. Arch. Allergy Appl. Immun., 81, 214-223), and applying it to a rabbit.

The above membrane was washed with PBS containing 0.05% Tween 20, and was reacted with a peroxidase-labelled anti-rabbit-IgG goat antibody (produced by Amersham Co.) diluted by a factor of 300 with PBS containing 1% bovine serum albumin. Then, the membrane was washed with PBS containing 0.05% Tween, and washed once with TBS (Tris-buffered saline). The membrane was stained by addition of 4-chloro-1-naphthol (produced by Bio-Rad Co.). Through the above operations, the intended recombinant protein was confirmed to be present in the culture supernatant and/or the Sf9 cells.

### (iv) Purification of recombinant virus:

After the above confirmation of the expression of the recombinant protein, the recombinant virus was purified by plaque formation to obtain a recombinant protein in high purity.

For the purification, 1.5 × 10⁶ cells of Sf9 was transferred preliminarily to a Petri dish of 60 mm diameter, and the culture medium was removed therefrom. Separately, the above culture supernatant was subjected to centrifugation at 700 × g for 10 minutes, and the resulting supernatant was diluted suitably. One milliliter of the diluted virus solution was added to the above-prepared Sf9 cells in a Petri dish.

A virus infection treatment was conducted with occasional shaking of the Petri dish, at room temperature for one hour. Thereafter, the diluted virus solution was removed. From the peripheral end of the Petri dish, 3 ml of an agarose solution was poured gently which had been prepared by subjecting a 3% LMP Agarose Ultra Pure (produced by BPL Co.) to an autoclave treatment and adding thereto a double volume of a bovine fetal serum-containing culture medium. The mixture was left standing at 27°C for 4 days. The formed plaques were selected appropriately for purification of the recombinant virus, and the purified recombinant virus was allowed to infect Sf9 cells to confirm the expression by the infection. This procedure was repeated several times to complete the purification of the recombinant virus.

### (3) Expression and Purification of Recombinant Protein (Precursor of Der f I Allergen):

### (i) Expression of recombinant protein:

The solution of the recombinant virus purified as above was added to incubated Sf9 cells to give an M.O.I (multiplicity of infection) of 5 pfu/cell, and the mixture was left standing for one hour with occasional stirring to have the cells infected with the recombinant virus.

An excess of the recombinant virus solution was removed, and then the bovine fetal serum-containing culture medium was added thereto. After incubation at 27°C for 24 hours, the culture medium was replaced by a serum free culture medium. Then, the mixture was incubated at 27°C for about 98 hours to express a recombinant protein. The culture supernatant and the precipitated Sf9 cells were collected to obtain an expressed recombinant protein.

### (ii) Purification of recombinant protein:

The above-collected recombinant protein solution was centrifuged at 700 × g for 10 minutes, and the resulting supernatant was passed through a 0.45 µm membrane filter. The filtered supernatant was desalted by use of a Sephadex G-50 column (produced by Pharmacia Co.) having been equilibrated with a 20 mM Tris-HCl buffer solution (pH 8.0). The void fraction was passed through a DEAE-sephacel column (produced by Pharmacia Co.) having been equilibrated with the same buffer solution, and the adsorbed matter was eluted with NaCl solutions by a linear concentration gradient of from 0 M to 0.3 M.

A fraction containing the recombinant protein was desalted through a Sephadex G-50 column having been equilibrated with a 10 mM phosphate buffer solution (pH 7.2). Thereafter the void fraction was passed through a hydroxyapatite column (produced by Bio-Rad Co.) having been equilibrated with the above buffer solution. The passed fraction was concentrated by means of an ultrafiltration membrane of fractionation molecular weight of 5000 Da to complete the purification of the recombinant protein. The yield was 50 mg per liter of the recombinant virus solution.

### (iv) SDS-PAGE analysis:

The purified recombinant protein was separated by electrophoresis according to the method shown in "Tanpakushitsu, Koso no Kiso Jikkenho (Fundamental Experiment Technique for Proteins and Enzymes), (Nankodo)". The resulting bands were stained by PAGE-Blue 83 (produced by Fluka Co.). The molecular weight of the obtained recombinant protein ranged from about 34 kd to 36 kd, which was higher than 25 kd of naturel *Der f* I allergen.

Since natural *Der f* I allergen exhibits a certain protease activity, the recombinant protein was measured for protease activity, and was found to have an activity lower than that of the natural allergen. From these results, the recombinant protein is predicted to be a forerunner such as a pre-precursor and a precursor of the *Der f* I allergen.

### (v) Determination of amino acid sequence (N-terminal amino acid sequence):

With the above prediction, the purified recombinant protein was isolated by electrophoresis of SDS-PAGE technique and blotted onto a PVDF membrane (polyvinylidene fluoride membrane, produced by Millipore Co.). The band to be analyzed was cut out, and analyzed by a vapor phase sequencer (manufactured by Applied Biosystems Co.) to determine the amino acid sequence. The results are shown in Sequences Nos. 5 to 8 of Sequence Table.

Sequence Table shows that the obtained recombinant protein is a precursor of *Der f* I allergen, which is consistent with the above prediction.

### (vi) Measurement of RAST activity (allergic activity):

The RAST activity of the recombinant protein (*Der f* I allergen precursor) was measured by means of RAST EIA kit (produced by Pharmacia Co.). The incubation buffer, the washing buffer, the enzyme-binding anti-IgE antibody, the substrate solution, and the reaction-terminating reagent employed were the ones attached to the RAST EIA kit.

In the measurement, the precursor of the *Der f* I allergen was allowed to be adsorbed onto a filter paper sheet activated by cyanogen bromide, by standing overnight. It was washed once with 0.1 M NaHCO₃. Thereto was added 1 M β-ethanolamine (pH 9.0), and the filter paper sheet was left standing at room temperature for 3 hours. Then it was washed once with 0.1 M NaHCO₃, three times with 0.1 M acetate buffer solution (pH 4.0), and twice with the incubation buffer solution. A human anti-mite IgE serum was added thereto, followed by shaking at 37°C for 3 hours. Then, washing was conducted with the washing buffer three times. Thereto was added the enzyme-binding anti-IgE antibody, and a reaction was allowed to take place at room temperature overnight.

Washing with the washing buffer was conducted three times; a substrate solution was added; and the mixture was shaken at 37°C for 2 hours to give rise to a reaction. Then, the reaction was stopped by addition of a reaction-terminating reagent, followed by measurement of OD₄₂₀ value. The results are shown in Fig 2 (see p-*Der f* I). The data of the natural *Der f* I allergen is shown also therein (see n-*Der f* I).

As shown in Fig. 2, the precursor of the *Der f* I allergen of the present invention has a significant allergic activity although it is less active than the natural *Der f* I allergen. A substance (protein) having such a level of RAST activity is considered to be reactive with the anti-mite IgE of a mite-allergic diseases patient.

### (3) Production of Der f I Allergen and Measurement of Its Allergic Activity:

### (i) pH adjustment and SDS-PAGE analysis:

A *Der f* I allergen was obtained by adjusting the pH of the above-prepared *Der f* I allergen precursor solution. Specifically, the *Der f* I allergen precursor solution was concentrated to about 1/10 volume by means of an ultrafiltration membrane (produced by Amicon Co.) of fractionation molecular weight of 5000 Da. The concentrated solution was diluted with an acetate buffer solution of a suitable concentration to the original volume. The operation was repeated several times. Thereby, solutions of the *Der f* I allergen precursors having different pH levels were obtained.

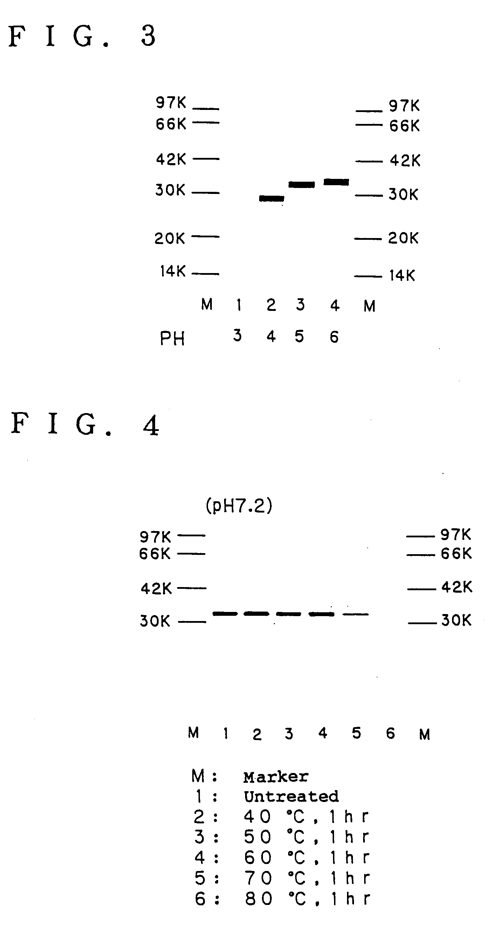

The solutions having different pH values were respectively subjected to electrophoresis according to the SDS-PAGE technique. Fig. 3 shows the results.

As shown in Fig. 3, after standing at a pH of about 4 at about 4°C for 2 days, the resulting substance had a molecular weight nearly equal to that of natural *Der f* I allergen. Presumably, the pH adjustment caused cleavage of the pro-sequence of the *Der f* I allergen precursor to form a *Der f* I allergen. As above, the solution adjusted to a pH of about 4 gave, in about two days, a substance having a molecular weight nearly equal to that of natural *Der f* I allergen. Meanwhile, a solution of a pH of about 4.4, when allowed to stand at about 4°C for about 30 days, gave a similar molecular weight change.

### (ii) Determination of amino acid sequence (N-terminal amino acid sequence):

On the above presumption, the *Der f* I allergen precursor which had be controlled to have a molecular weight nearly equal to that of natural *Der f* I allergen, was subjected to the same operation as in the above Item (2), (v) to determine the amino acid sequence. The results are shown by Sequence No. 1 and Sequence No. 2 in Sequence Table.

As shown in Sequence Table, the obtained substance was *Der f* I allergen (recombinant *Der f* I allergen). This shows that the pro-sequence was cleaved by the above pH adjustment, and is consistent with the above presumption. The resulting recombinant *Der f* I allergen was a mixture of a protein represented by Sequence No. 1 and a protein represented by Sequence No. 2, and the mixing ratio was typically 50% : 50%.

### (iii) Measurement of RAST activity:

The obtained recombinant *Der f* I allergen was measured for RAST activity in the same operation as in the above Item (2)-(vi). The result is shown in Fig. 2 (see r-*Der f* I).

As shown in Fig. 2, the obtained recombinant *Der f* I allergen had a RAST activity nearly equal to that of natural *Der f* I allergen.

### (iv) Investigation of influence of pH, temperature and treating time on activity:

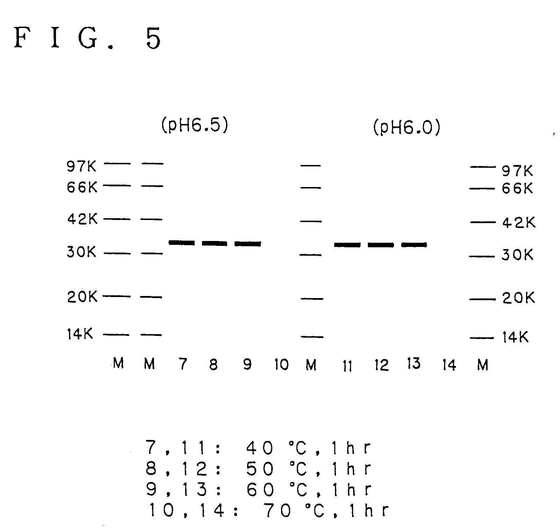

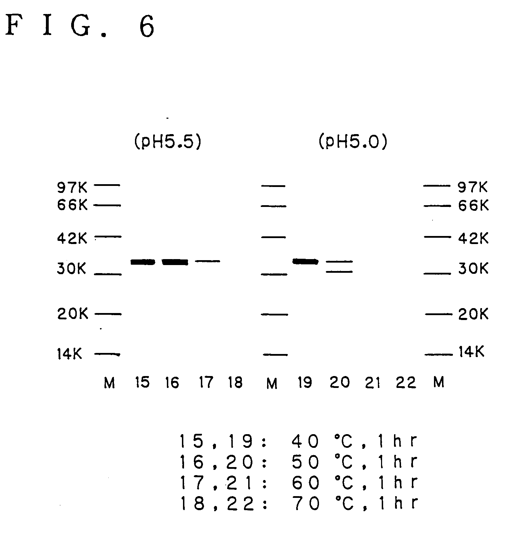

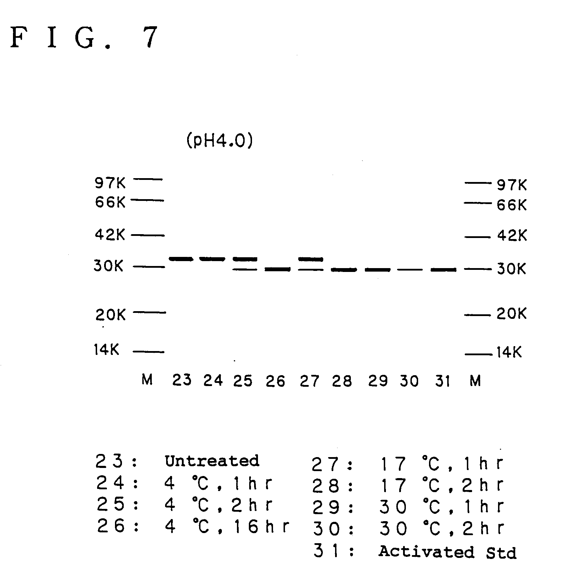

The above *Der f* I allergen precursor solution was treated as above by using a citric acid-disodium hydrogenphosphate buffer solution in place of the acetate buffer solution and changing the pH, the temperature, and the treating time. The treated product was analyzed by SDS-PAGE technique. The results are shown in Figs. 4 to 7. The influence on the activation is summarized in Table 2. In Table 2, the treating time was one hour unless specially mentioned.

**Table 2**

| Influence of pH, Temperature, and Treating Time on Activation | | | | | | |
|---|---|---|---|---|---|---|
| Temperature | pH | | | | | |
| | 7.2 | 6.5 | 6.0 | 5.5 | 5.0 | 4.0 |
| 4°C | - | - | - | - | - | ○ *1 |
| 17°C | - | - | - | - | - | △ *2 |
| 30°C | - | - | - | - | - | ○ |
| 40°C | X | X | X | X | X | - |
| 50°C | X | X | X | X | △ | - |
| 60°C | X | X | X | X, dec | disapp | - |
| 70°C | X, dec | X, dec | disapp | disapp | disapp | - |
| 80°C | X | X | X | - | - | - |
| Evaluation: ○: Activated, △: Activated by about half, X: Not activated, dec: Protein decreased, disapp: Protein disappeared | | | | | | |

| | | | | | | |
|---|---|---|---|---|---|---|
| *1: Treated for 16 hours | | | | | | |
| *2: Treated for 2 hours | | | | | | |

As shown in Figs 4 to 7 and Table 1, the activation does not occur at pH in the neutral range even with heating. That is, the heating is not an important influential factor in the neutral range of about pH 7.

On the other hand, at pH 6.0, the band in SDS-PAGE disappeared at 70°C, and at pH 5.0, the band disappeared at 60°C. Therefore, *Der f* I allergen is considered to be unstable under weakly acidic and high-temperature conditions.

Accordingly, it is thought that the activation is preferably conducted at a relatively low temperature.

### (v) Preparation of Der f I allergen by site-specific variation and with site-specific protease, and measurement of allergic activity:

A DNA molecule (derived from a natural product) for encoding the amino acid sequence of *Der f* I allergen was treated by a PCR method to change the codon for encoding C-terminal amino acids of the pro-sequence into a codon for encoding lysine. In this codon change, the codon for encoding the C-terminal asparagine of the pro-sequences of Sequence Nos. 5 and 7, and the codon for encoding the C-terminal glutamic acid of the pro-sequences of Sequence Nos. 6 and 8 were respectively transformed into a codon for encoding lysine in order to develop finally the *Der f* I allergen represented by Sequence Nos. 1 and 2.

The resulting transformed DNA molecules were respectively expressed by utilizing Sf9 cells as described above to obtain variant precursors of the recombinant *Der f* I allergen.

Of the obtained variant precursors, the one of Sequence No. 5 or 7 having, at the C-terminal, lysine in place of asparagine is called herein as "variant precursor (-3L)", and another one of Sequence No. 6 or 8 having, at the C-terminal, lysine in place of glutamic acid is called herein as "variant precursor (-1L)".

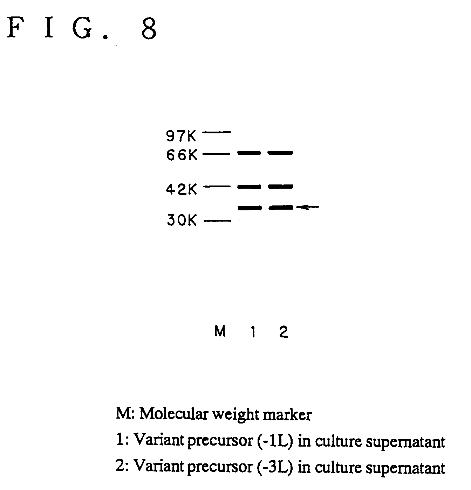

The obtained variant precursors (-3L) and (-1L) were analyzed by the SDS-PAGE technique. Fig. 8 shows the results.

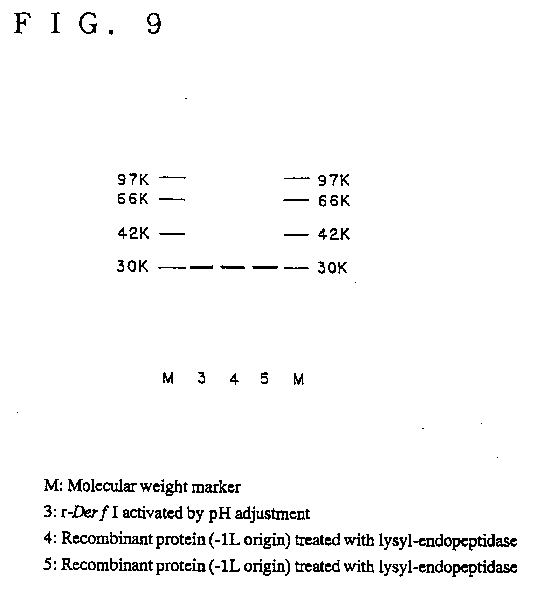

The variant precursors (-3L) and (-1L) were reacted with lysyl-endopeptidase, a site-specific protease. The resulting recombinant proteins (-3L origin) and (-1L origin) were analyzed by the SDS-PAGE technique. Fig. 9 shows the results. Fig. 9 shows additionally the analysis result of the recombinant *Der f* I allergen activated by the above pH adjustment as the reference.

As shown in Fig. 9, the recombinant proteins (-1L origin) and (-3L origin) had respectively a molecular weight nearly equal to that of the recombinant *Der f* I allergen obtained by the above pH adjustment. Accordingly, the recombinant protein (-1L origin) is considered to correspond to the recombinant *Der f* I allergen represented by Sequence No. 2, and the recombinant protein (-3L origin) is considered to correspond to the recombinant *Der f* I allergen represented by Sequence No. 1.

RAST activities were measured for natural *Der f* I allergen and the above obtained substances, i.e. the recombinant *Der f* I precursor, the *Der f* I obtained by pH adjustment, the variant precursor (-1L), the variant precursor (-3L), the recombinant protein (-1L origin) and the recombinant protein (-3L origin). Table 10 shows the results.

Table 3 summarizes the degrees of activation of the recombinant *Der f* I precursor and the variant precursors [(-1L) and (-3L)].

**Table 3**

| | Activation degree (%) *¹ | |
|---|---|---|
| | Before treatment | After treatment |
| Recombinant *Der f* I precursor | 10 | Not less than 100 *² |
| Variant precursor (-1L) | 10 | Not less than 100 *³ |
| Variant precursor (-3L) | 10 | Not less than 100 *³ |

| | | |
|---|---|---|
| *1 Calculated on the basis of IgE-binding activity of natural *Der f* I (100%) | | |
| *2 Activation treatment by pH adjustment | | |
| *3 Activation treatment by lysyl endopeptidase | | |

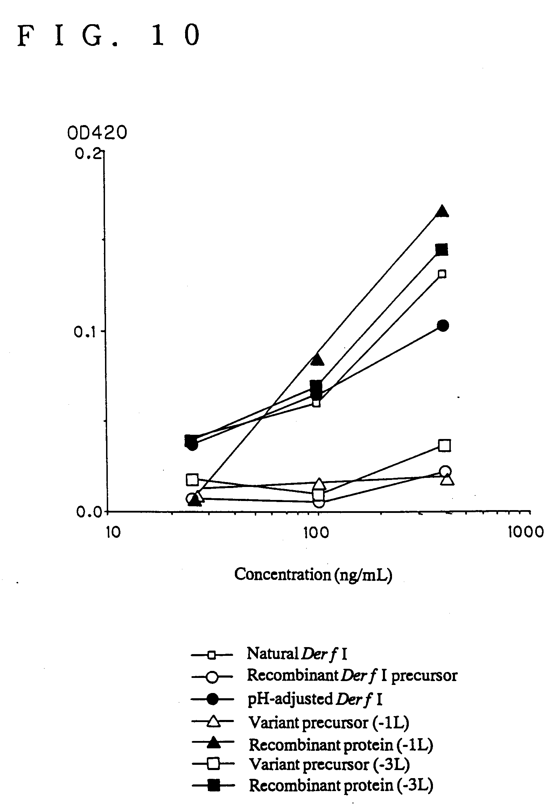

Fig. 10 and Table 3 show that the special post-treatment of the *Der f* I precursor and the variant precursors gave recombinant *Der f* I allergens each having the same level of allergic activity as the natural *Der f* I allergen.

The site-specific variation and the activation with lysyl-endopeptidase are based on the findings by the inventors of the present invention that the lysyl-endopeptidase cuts only the lysine group portion of the pre-pro-sequence of *Der f* I allergen but does not cut the lysine group portion of the matured sequence. Such facts have not ever been anticipated, and are surprising.

### (vi) Activation of variant precursors (-1L) and (-3L) by pH adjustment:

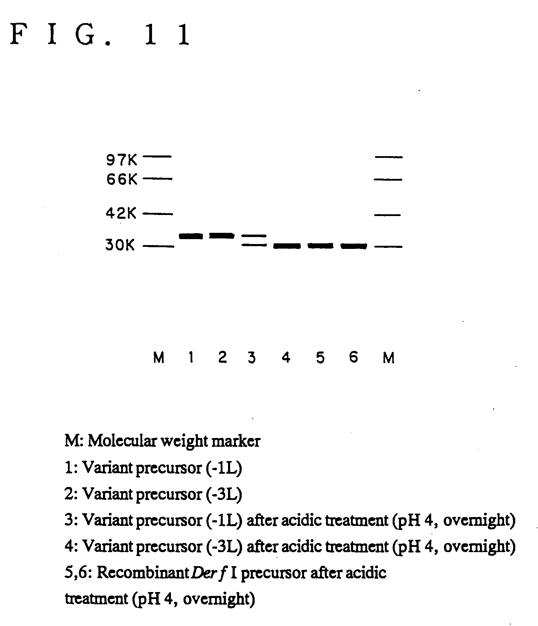

The variant precursors (-1L) and (-3L) prepared as above were adjusted to be at pH 4 by the procedure of Item (i) above and left standing overnight, and then were subjected to SDS-PAGE analysis. Fig. 11 shows the results. Incidentally, the activation was complete in 5 days.

Fig. 11 shows that the variant precursor (-3L) was activated by pH adjustment, but the variant precursor (-1L) was activated at a lower rate even though it was activatable. Based on this fact, if the technique of precursor modification by replacement of the C-terminal glutaminic acid of the pro-sequence represented by Sequence No. 6 or 8, with another amino acid and subsequent pH adjustment of the resulting variant precursor is applied to the desentization therapy, the development of allergy activity would be delayed to mitigate the shock of the allergen-administered patient.

In the above, the present invention has been described by way of the preferred embodiments. However, the present invention is not limited thereto, and may be variant within the gist of the present invention.

For instance, the present invention can readily be applied to allergens of Group I, particularly to *Der p* I. Further, the present invention would be applicable not only to the allergens of Group I but also to the allergens of Group II. Furthermore, the present invention would be applicable not only to *Der f* and *Der p* but also to allergens originated from *Der m* (*Dermatophagoides microceras*).

### INDUSTRIAL APPLICABILITY

As described above, in the present invention, genetic recombination is conducted in insect cells, and a certain post-treatment is conducted as desired. Thereby the present invention provides a novel precursor of a recombinant *Der* I allergenic substance, which precursor can be secreted from cells and is in a solution form; a novel recombinant *Der* I allergenic substance having an allergenic activity equal to that of natural product; and a process for producing the above substances at a high yield.

Accordingly the present invention makes feasible use of hyposensitization therapy for allergenosis, in particular atopic asthma, which has become a problem in recent years.

SEQUENCE TABLE
Sequence No.: 1
Sequence length: 225
Sequence type: Amino acid
Strand number: Single strand
Topology: Linear
Kind of sequence: Peptide
Origin, kind of cells: Dermatophagoides farinae
Sequence

Sequence No.: 2
Sequence length: 223
Sequence type: Amino acid
Strand number: Single strand
Topology: Linear
Kind of sequence: Peptide
Origin, kind of cells: Dermatophagoides farinae
Sequence

Sequence No.: 3
Sequence length: 321
Sequence type: Amino acid
Strand number: Single strand
Topology: Linear
Kind of sequence: Peptide
Origin, kind of cells: Dermatophagoides farinae
Sequence

Sequence No.: 4
Sequence length: 321
Sequence type: Amino acid
Strand number: Single strand
Topology: Linear
Kind of sequence: Peptide
Origin, kind of cells: Dermatophagoides farinae
Sequence

Sequence No.:          5
Sequence length:       303
Sequence type:         Amino acid
Strand number:         Single strand
Topology:              Linear
Kind of sequence:      Peptide
Origin, kind of cells: Dermatophagoides farinae
Sequence

Sequence No.: 6
Sequence length: 303
Sequence type: Amino acid
Strand number: Single strand
Topology: Linear
Kind of sequence: Peptide
Origin, kind of cells: Dermatophagoides farinae
Sequence

Sequence No.: 7
Sequence length: 282
Sequence type: Amino acid
Strand number: Single strand
Topology: Linear
Kind of sequence: Peptide
Origin, kind of cells: Dermatophagoides farinae
Sequence

Sequence No.: 8
Sequence length: 282
Sequence type: Amino acid
Strand number: Single strand
Topology: Linear
Kind of sequence: Peptide
Origin, kind of cells: Dermatophagoides farinae
Sequence

## Claims

1. A recombinant *Der f* I allergenic substance composed of one or more proteins selected from the group consisting of proteins having an amino acid sequence represented by Sequence No. 1 or Sequence No. 2 of Sequence Table.

2. The recombinant *Der f* I allergenic substance of claim 1, which is reactive to anti-mite IgE of mite-allergic diseases patient.

3. A process for production of a recombinant *Der* I allergenic substance, which comprises expressing a *Der* I gene in insect cells by genetic recombination and bringing the pH of the resulting solution of a recombinant *Der* I allergen precursor to an acidic side.

4. The process of claim 3, wherein the recombinant *Der* I allergenic substance is the recombinant *Der f* I allergenic substance as set forth in claim 1 or a recombinant *Der p* I allergenic substance.

5. A recombinant *Der f* I allergenic substance precursor, composed of one or more proteins selected from the group of proteins having an amino acid sequence represented by Sequence No. 5, 6, 7, or 8 of Sequence Table.

6. The recombinant *Der f* I allergenic substance precursor of claim 5, which is reactive to anti-mite IgE of mite-allergic diseases patient.

7. A process for production of a recombinant *Der f* I allergenic substance precursor of claim 5, which comprises expressing a *Der f* I gene in insect cells by genetic recombination technique.

8. A process for production of a recombinant *Der* I allergenic substance, which comprises:
inducing site-specific variation in a DNA molecule for encoding a *Der* I allergenic substance to form a DNA molecule of *Der* I having a recognition sequence for a site-specific protease,
expressing the DNA molecule in insect cells to form a *Der* I allergenic substance precursor having the above recognition sequence, and then
reacting said precursor with a corresponding site-specific protease.

9. The process of claim 8, wherein the recombinant *Der* I allergenic substance is the recombinant *Der f* I allergenic substance as set forth in claim 1 or a recombinant *Der p* I allergenic substance.
